# EUROPEAN PATENT APPLICATION

(11) **EP 1 808 131 A1**
(43) Date of publication of application: **18.07.2007**
(21) Application number: 05790554.9
(22) Date of filing: 05.10.2005
(51) Int. Cl.: A61B 8/12

(54) **ELECTRONIC RADIAL TYPE ULTRASONIC VIBRATOR, AND ULTRASONIC ENDOSCOPE AND ITS MANUFACTURING METHOD**

(30) Priority: 15.10.2004 JP 2004301294; 26.01.2005 JP 2005018481
(71) Applicant: OLYMPUS MEDICAL SYSTEMS CORP., Shibuya-ku Tokyo 151-0072 (JP); Olympus Corporation, Tokyo 151-0072 (JP)
(72) Inventor: WAKABAYASHI, Katsuhiro, 1920043 (JP); SAWADA, Yukihiko, 3420041 (JP); MIZUNUMA, Akiko, 1920065 (JP); IMAHASHI, Takuya, Kawasaki-shi, Kanagawa 2140006 (JP); SATO, Sunao, 1920032 (JP)
(74) Representative: von Hellfeld, Axel
(86) International application number: PCT/JP2005/018430
(87) International publication number: WO 2006/040972

(57) **Abstract**

An electronic radial type ultrasonic transducer, in which electrode pads to be electrically connected to signal wires are formed on a printed circuit board, forms the electrode pads on the top surface of the printed circuit board in a radial pattern and also exposes the electrode pads relative to the outer circumference of the printed circuit board, configures a wire by coating, in a predetermined thickness, the entirety of a substrate constituting the substrate of the wire with a different kind of conductive metal that is different from the substrate, and melts the different kind of metal at both ends of the wire, thereby electrically connecting the electrode pads to the ultrasonic transducer by way of the wire and electrically connecting the ultrasonic transducer to the signal wires.

## Description

### Technical Field

The present invention relates to an ultrasonic endoscope using an electronic radial type ultrasonic transducer.

### Background Art

An electronic radial type ultrasonic endoscope is an endoscope electrically scanning an ultrasonic beam by using a plurality of piezoelectric elements equipped at the head of an insertion part (piezoelectric elements are equipped in the outer circumferential direction of the insertion part) of the endoscope. Further describing it, a transmission and reception of an ultrasonic beam in the direction perpendicular to the insertion direction of the endoscope makes it possible to obtain an ultrasonic image. It is known that a combination use of such an ultrasonic endoscope and an optical endoscope makes it possible to correlate an ultrasonic image with an optical image, and accordingly ease a diagnosis.

Meanwhile, also known is a use of a printed circuit board for connecting piezoelectric elements for an electronic radial ultrasonic endoscope in order to ease a wiring with a cable.

Related to a printed circuit board for electrically connecting to piezoelectric elements that are arrayed in a donut shape, disclosed is a technique of a printed circuit board for electrically connecting a donut shaped board that comprises a signal pattern for electrically connecting to the piezoelectric elements to a signal pattern corresponding to the respective signal patterns (e.g., refer to a patent document 1).

The piezoelectric elements of the electronic radial ultrasonic endoscope are built within a head hard part of the endoscope with a piezoelectric element constituting a reference being positioned at a specific position in order to correlate between an optical image and an ultrasonic image.

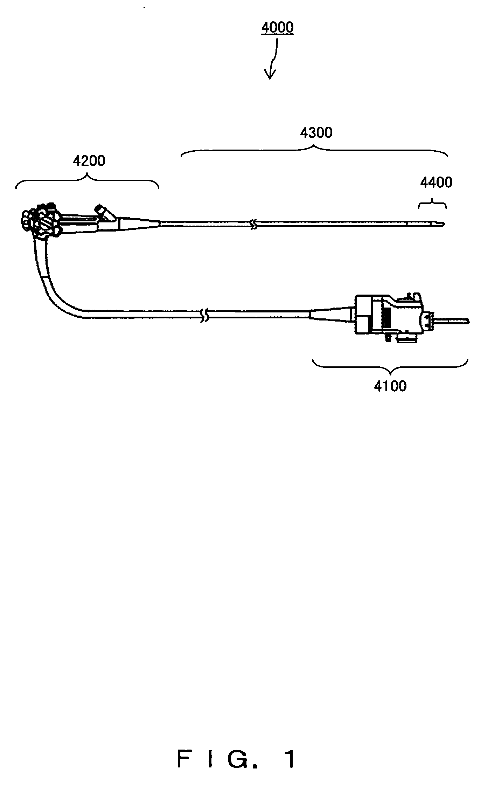

Fig. 1 is a diagram showing a conventional ultrasonic endoscope.

The electronic radial ultrasonic endoscope 4000 shown by Fig. 1 comprises a connection part 4100, an operation part 4200, and an insertion part 4300 that comprises a head part 4400.

The connection part 4100 is connected to such as a ultrasonic diagnostic equipment for example comprising a display, and an image obtained by an ultra-compact camera or such equipped on the head part 4400 is displayed in the display.

The operation part 4200 performs a curving operation in the up, down, left and right directions for example of the insertion part 4300 by an operation of a user.

The head part 4400 is equipped with an ultrasonic transducer array constituted by a plurality of ultrasonic transducers for example being continuously arrayed in addition to the ultra-compact camera, and an ultrasonic wave is emitted and received at a selected ultrasonic transducer(s) selected from among the plurality thereof comprised by the ultrasonic transducer array. An ultrasonic received by the ultrasonic transducer array is converted into an electric signal and displayed in the display, or such, as an image.

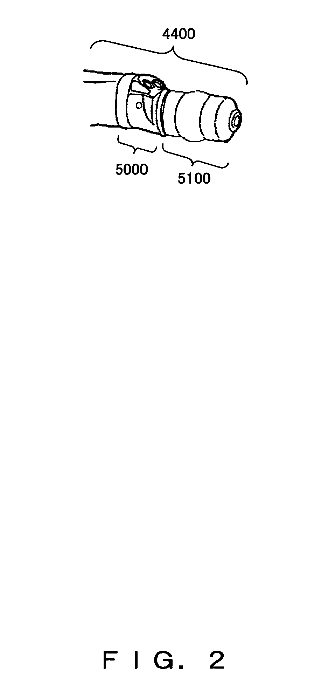

Fig. 2 is an enlarged diagram of the head part 4400 shown in Fig. 1.

As shown in Fig. 2, the head part 4400 comprises a camera part 5000 equipped with an ultra-compact camera, an illumination and such, and an ultrasonic part 5100 equipped with the ultrasonic transducer array and such.

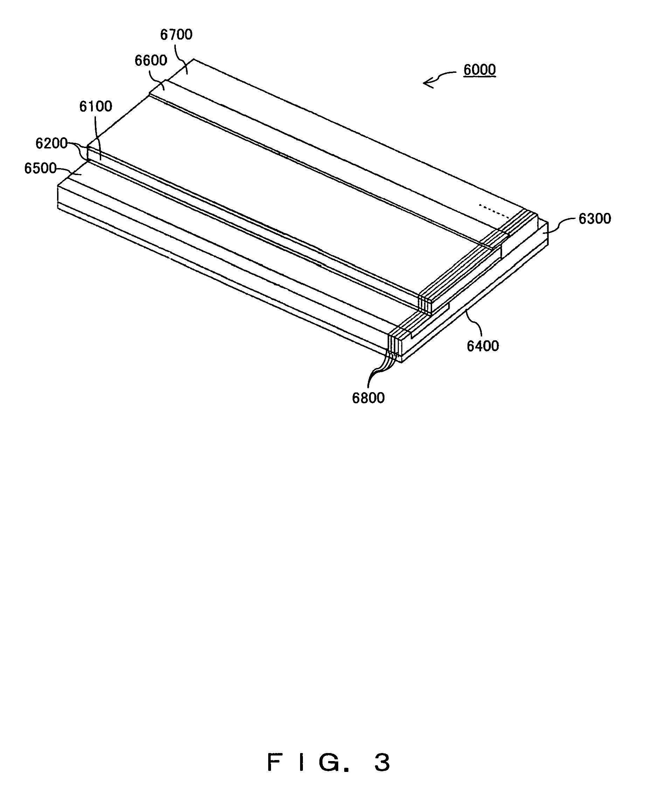

Fig. 3 is a diagram showing an example of an ultrasonic transducer array. Note that the ultrasonic transducer array shown in Fig. 3 shows only a fundamental comprisal.

As shown in Fig. 3, the ultrasonic transducer array 6000 comprises a piezoelectric element 6100, electrodes 6200, a first acoustic matching layer 6300, a second acoustic matching layer 6400, a conductive resin 6500, a conductive body 6600 and a substrate 6700.

The piezoelectric element 6100, electrodes 6200, first acoustic matching layer 6300, second acoustic matching layer 6400, conductive resin 6500, conductive body 6600 and substrate 6700 are divided into a plurality of parts by grooves 6800 that are commonly featured, thereby comprising the plurality of ultrasonic transducers.

The individual ultrasonic transducer are electrically connected to corresponding signal wires respectively, so that an ultrasonic transducer emits an ultrasonic wave when a signal is transmitted from a measurement apparatus or such to the ultrasonic transducer by way of a signal wire. Conversely, receiving an ultrasonic wave, the ultrasonic transducer converts the ultrasonic wave into an electric signal and sends it to the measurement apparatus and such by way of a signal wire.

Meanwhile, side faces of the ultrasonic transducer array 6000 in the direction perpendicular to the longitudinal direction thereof are connected together to form the ultrasonic transducer array 6000 in a cylindrical form, thereby configuring a radial type ultrasonic transducer array that emits an ultrasonic wave in a circular pattern.

Among such configured radial type ultrasonic transducer arrays, some is configured to connect an ultrasonic transducer and a signal wire together at the head part of the ultrasonic transducer array. Also, among such configured radial type ultrasonic transducer arrays connecting the ultrasonic transducer and signal wire together at the head part, some is configured to connect the ultrasonic transducer and a signal wires together by way of the substrate at the head part of a cylindrical structure member internally assembled within the ultrasonic transducer array.

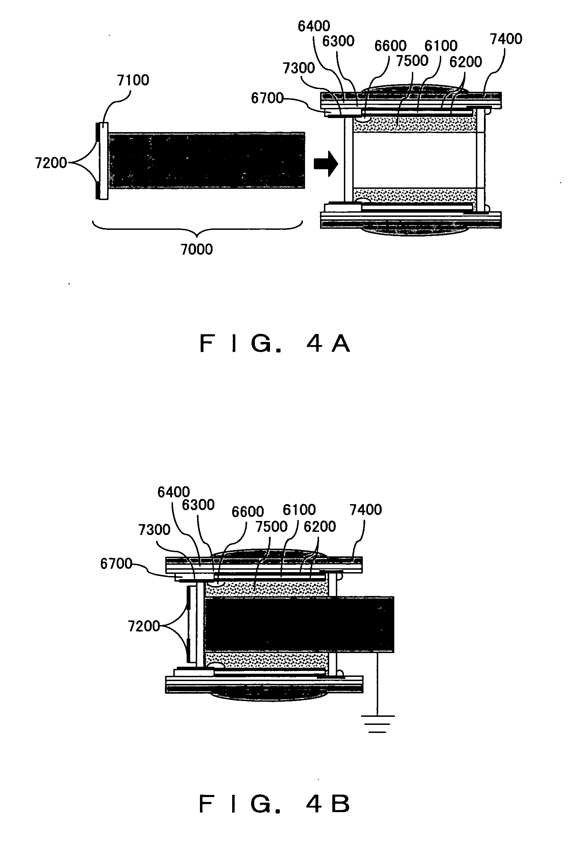

Fig. 4A is a diagram showing how a cylindrical structure member is assembled to a radial type ultrasonic transducer array; and Fig. 4B is a diagram showing a radial type ultrasonic transducer array assembled with a cylindrical structure member. Note that the same component sign is assigned to the same configuration as one shown in Fig. 3.

As shown in Figs. 4A and 4B, the head part of the cylindrical structure member 7000 is equipped with a board 7100 on which a plurality of pads 7200 respectively corresponding to the plurality of ultrasonic transducers are equipped. The individual substrates 6700 of the plurality of ultrasonic transducers are respectively equipped with conductive layers 7300. An outer circumference of the radial type ultrasonic transducer array is equipped with an acoustic lens 7400. And an inner circumference of the radial type ultrasonic transducer array is equipped with a backing material 7500.

The first step inserts the cylindrical structure member 7000 into the radial type ultrasonic transducer array as shown in Fig. 4A. The assumption here is that the individual pads 7200 featured on the board 7100 are electrically connected to the respective signal wires in advance of this process.

The above process results in assembling the cylindrical structure member 7000 with the radial type ultrasonic transducer array as shown in Fig. 4B. After assembling the cylindrical structure member 7000 with the radial type ultrasonic transducer array, the individual pads 7200 featured on the board 7100 for example are electrically connected to the respective conductive layers 7300 by way of the conductive wires.

As a result of this process, the individual ultrasonic transducers and respective signal wires are electrically connected to each other by way of the pads 7200, wires, conductive layers 7300, conductive members 6600 and electrodes 6200.

As such, a configuration electrically connecting the signal wire to ultrasonic transducer by way of the board makes an easy positioning between the ultrasonic transducers and signal wires, thus making a wiring work between the ultrasonic transducers and signal wires smooth (e.g., refer to a patent document 2 or 3).

As for a wiring method for connecting a signal wire to a board, known are a wire bonding, et cetera, for example (e.g., refer to a patent document 4). Incidentally, when connecting wires to the respective ultrasonic transducers of the radial type ultrasonic transducer array, it is very difficult to connect a wire to the pad 7200 by means of a wire bonding for example because the individual ultrasonic transducers are lined up in a circular pattern.

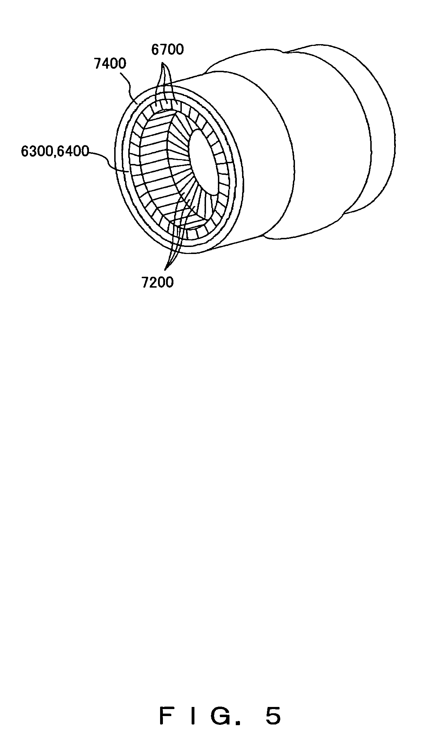

Fig. 5 is a diagram showing a radial type ultrasonic transducer array assembled with the cylindrical structure member 7000. Note that the same component sign is assigned to the same configuration as one shown in Figs. 4A and 4B.

Referring to Fig. 5, the pads 7200 and substrates 6700 having conductive layers corresponding to the pads 7200 on the surface thereof are desirably positioned with each other as accurate as possible, and an accurate positioning of the pads 7200 against the substrates 6700 having the conductive layers on the surface makes a wiring work of the wires easy, hence further easing a wiring work between the ultrasonic transducers and signal wires.

Incidentally, the head part of an ultrasonic endoscope is desired to be as small a diameter as possible, and therefore, not only a radial type ultrasonic transducer array, but an ultrasonic transducer array incorporated therein is desirably configured as compact as possible.

Consequently, the individual ultrasonic transducers used for the ultrasonic transducer array incorporated into the ultrasonic endoscope is lined up in a very narrow pitch, making the wiring work of wires very difficult. As an example, in the case of incorporating a radial type ultrasonic transducer array constituted by 200 pieces of ultrasonic transducers in the head part 4400 of a diameter of 10 mm, these ultrasonic transducers are lined up in no more than 0.2 mm pitch.

There is a method of providing a backup solder at the tip part of the wire for example as a method for making the wiring work of the wire smooth in the case of individual ultrasonic transducers being lined up in a very narrow pitch.

In the case of providing the tip part of the wire with a backup solder, a common method is to apply a small amount of solder to the tip part of the wire by using such as a soldering iron or to dip the tip part of the wire in a solder bath; these methods, however, may not always be capable of providing a uniform amount of solder at the tip part of the respective wires. Consequently, if there is a variation in the amount of a backup solder being provided on the tip parts of the respective wires, there is a risk of the applied solders on the wires lined up adjacent to one another sticking together to form a bridge when connecting the respective wires to a plurality of ultrasonic transducers.

Such is a problem of difficulty in wiring work of the wires when connecting the ultrasonic transducers and signal wires together by way of the wires in the case of a plurality of ultrasonic transducers being lined up in a very narrow pitch.

Likewise, an ultrasonic probe noted in the patent document 1 has been faced with a problem of a difficulty in validating an electrical continuity because a pad pitch is very narrow when validating the continuity on a board pattern electrically connecting to a donut shaped board by using a probe used for a continuity validation. That is, the pad pitch is very narrow as a result of shrinking a size of the donut shaped board in the radial direction in order to make the diameter of the insertion part of an ultrasonic probe as small as possible.

This requires a use of a sharp-pointed probe used for validating an electrical continuity when validating the electrical continuity. As a result, there has been a possibility of not being able to secure the continuity eventually even if it is validated because a pad or connecting part between the pad and cable may have been damaged in the validation process.

The ultrasonic probe noted in the patent document 1 has also been faced with a possible risk of a wrong connection when the donut shaped board and piezoelectric elements are electrically connected together by a wire bonding because of a marking not provided for indicating a reference at the time of the electrical connection.

Meanwhile, when assembling an endoscope by positioning a piezoelectric element constituting a reference to a specific position in order to correlate an optical image with an ultrasonic image, another piezoelectric element may be wrongly chosen as the piezoelectric element constituting a reference, resulting in causing a problem of creating a shift in a correlation between the optical image and ultrasonic image.

In consideration of the above problem, the present invention aims at providing an electronic radial type ultrasonic transducer not causing damage to a pad or a connection part of the pad and cable when validating an electrical continuity by using a probe used for an electrical continuity validation.

The present invention also aims at providing an electronic radial type ultrasonic transducer employing a printed circuit allowing no wrong connection when electrically connecting a piezoelectric element to a pad of a printed circuit board connected to a cable, nor a displacement between an optical image and an ultrasonic image.

The present invention further aims at providing a production method for an ultrasonic transducer array and its wire for making a wiring work easy when connecting ultrasonic transducers and signal wires together by way of the wires even if a plurality of ultrasonic transducers are lined up in a very narrow pitch.

Patent document 1: Laid-Open Japanese Patent Application Publication No. H08-172695

Patent document 2: Registered Japanese Patent No. 2729442

Patent document 3: Laid-Open Japanese Patent Application Publication No. 2003-33354

Patent document 4: Laid-Open Japanese Patent Application Publication No. H03-254737

### Disclosure of Invention

In order to solve the above noted problems, the present invention has adopted the following comprisals.

That is, an electronic radial type ultrasonic transducer according to the present invention is one arraying a plurality of piezoelectric elements for transmitting a drive signal driving each piezoelectric element in a circular pattern for emitting and receiving an ultrasonic wave, storing cables corresponding to each of the piezoelectric elements in the inside of a cylindrical body formed thereby, forming electrode pads electrically connecting the cables and piezoelectric elements together on a printed circuit board, featuring a hole at the center of the printed circuit board in the planar direction for letting the cables go through and equipping the printed circuit board on the cylindrical body, wherein the electrode pads are formed on the top surface of the printed circuit board in a radial pattern and also exposed relative to an outer circumference of the printed circuit board.

The electronic radial type ultrasonic transducer may also be configured to form a groove in a shorter direction of the outer circumference of the printed circuit board, of which a part contacts with the electrode pads.

The electronic radial type ultrasonic transducer may also be configured such that at least one electrode pad of the plurality thereof is in a different shape or size from other electrode pads.

The electronic radial type ultrasonic transducer may also be configured such that the printed circuit board related to both sides of at least one electrode pad of the plurality of electrode pads is equipped with a cutoff and a guide member is equipped at a predetermined position in the inside of the cylindrical body in order to guide the printed circuit board along the cutoff for installing the printed circuit board.

The electronic radial type ultrasonic transducer may also be configured to provide a marking on the top or side surface of the printed circuit board, or on a side surface of a support member supporting the printed circuit board, for identifying the electrode pad electrically connecting to a predetermined piece of the piezoelectric elements.

Also, the present invention extends to an ultrasonic endoscope comprising the electronic radial type ultrasonic transducer.

Also, an ultrasonic transducer array according to the present invention is one comprising a plurality of ultrasonic transducers continuously lined up and a plurality of signal wires electrically connected to the plurality of ultrasonic transducers respectively, comprising a plurality of wires respectively equipped between the plurality of ultrasonic transducers and the plurality of signal wires for electrically connecting the plurality of ultrasonic transducers and the plurality of signal wires respectively, wherein each of the plurality of wires is constituted by coating, in a predetermined thickness, the entirety of a metal constituting a substrate of the wire with a conductive metal that is different kind from the substrate, and the different kind of metal is melted at both ends of the respective wires, thereby electrically connecting the plurality of signal wires to the plurality of ultrasonic transducers respectively.

The wire comprised by the ultrasonic transducer array is preferably configured by coating the entirety of the substrate with the different kind of metal of a thickness so as not to become bridged with the adjacent wire when the different kind of metals at both ends of the wires are respectively melted.

The wire comprised by the ultrasonic transducer array may also be configured such that the substrate is constituted by cupper or silver, and the different kind of metal is constituted by a metal of which the main component is lead, tin or bismuth.

The wire comprised by the ultrasonic transducer array may also be configured by coating the entirety of the substrate with the different kind of metal by means of a wet plating.

The wire comprised by the ultrasonic transducer array may also be configured such that the substrate is not covered with an insulator.

The wire comprised by the ultrasonic transducer array may also be configured such that the substrate is a rectangular wire.

The wire comprised by the ultrasonic transducer array may also be configured such that the end surfaces of both ends of the wire are not coated with the different kind of metal.

Meanwhile, a production method for a wire according to the present invention is one for the wire equipped between an ultrasonic transducer and a signal wire for electrically connecting the ultrasonic transducer and signal wire together for an ultrasonic transducer array that comprises a plurality of ultrasonic transducers continuously lined up and a plurality of signal wires electrically connected to the plurality of ultrasonic transducers respectively, configuring the wire by coating the entirety of a substrate constituting a substrate of the wire with different kind of metal from the substrate by means of a wet plating.

The wet plating of the production method for the wire may employ an electroplating or chemical plating, (electroless plating).

The production method for the wire may apply a wet plating to the substrate by winding the substrate onto a plurality of rods equipped on a fixing tool.

### Brief Description of Drawings

Fig. 1 is a diagram showing a conventional ultrasonic endoscope;
Fig. 2 is an enlarged diagram of a head part;
Fig. 3 is a diagram showing an example of an ultrasonic transducer array;
Fig. 4A is a diagram showing how a cylindrical structure member is assembled to a radial type ultrasonic transducer array;
Fig. 4B is a diagram showing a radial type ultrasonic transducer array assembled with a cylindrical structure member;
Fig. 5 is a diagram showing a radial type ultrasonic transducer array assembled with the cylindrical structure member;
Fig. 6 is a diagram showing an external configuration of an ultrasonic endoscope according to the present invention;
Fig. 7A is an enlarged diagram of a head part of the ultrasonic endoscope shown in Fig. 6;
Fig. 7B is an enlarged diagram of a head part of the ultrasonic endoscope shown in Fig. 6;
Fig. 8 is a diagram showing a production process (part 1) of an ultrasonic transducer;
Fig. 9A is a diagram showing a production process (part 2) of an ultrasonic transducer;
Fig. 9B is a diagram showing a production process (part 2) of an ultrasonic transducer;
Fig. 9C is a diagram showing a production process (part 2) of an ultrasonic transducer;
Fig. 10A is a diagram showing a production process (part 3) of an ultrasonic transducer;
Fig. 10B is a diagram showing a production process (part 3) of an ultrasonic transducer;
Fig. 10C is a diagram showing a production process (part 3) of an ultrasonic transducer;
Fig. 11A is a diagram showing a production process (part 4) of an ultrasonic transducer;
Fig. 11B is a diagram showing a production process (part 4) of an ultrasonic transducer;
Fig. 12 shows an upper cross-sectional diagram of the head of the electronic radial ultrasonic endoscope shown in Fig. 11B;
Fig. 13 shows a side cross-sectional diagram of the head of the electronic radial ultrasonic endoscope shown in Fig. 11B;
Fig. 14A shows a top face, and a side cross-section, of a printed circuit board according to a first embodiment;
Fig. 14B shows a top face, and a side cross-section, of a printed circuit board according to the first embodiment;
Fig. 14C shows a top face, and a side cross-section, of a printed circuit board according to the first embodiment;
Fig. 14D shows a top face, and a side cross-section, of a printed circuit board according to the first embodiment;
Fig. 14E shows a top face, and a side cross-section, of a printed circuit board according to the first embodiment;
Fig. 14F shows a top face, and a side cross-section, of a printed circuit board according to the first embodiment;
Fig. 15A shows a top face, and a side cross-section, of a printed circuit board according to a second embodiment;
Fig. 15B shows a top face, and a side cross-section, of a printed circuit board according to the second embodiment;
Fig. 15C shows a top face, and a side cross-section, of a printed circuit board according to the second embodiment;
Fig. 15D shows a top face, and a side cross-section, of a printed circuit board according to the second embodiment;
Fig. 16A shows a top face, and a side cross-section, of a printed circuit board according to a third embodiment;
Fig. 16B shows a top face, and a side cross-section, of a printed circuit board according to the third embodiment;
Fig. 16C shows a top face, and a side cross-section, of a printed circuit board according to the third embodiment;
Fig. 16D shows a top face, and a side cross-section, of a printed circuit board according to the third embodiment;
Fig. 16E shows a top face, and a side cross-section, of a printed circuit board according to the third embodiment;
Fig. 16F shows a top face, and a side cross-section, of a printed circuit board according to the third embodiment;
Fig. 17A shows a top face of a printed circuit board according to a fourth embodiment;
Fig. 17B shows a top face of a printed circuit board according to the fourth embodiment;
Fig. 17C shows a top face of a printed circuit board according to the fourth embodiment;
Fig. 17D shows a top face of a printed circuit board according to the fourth embodiment;
Fig. 18A shows a top face, and diagonal view, of a printed circuit board according to a fifth embodiment;
Fig. 18B shows a top face, and diagonal view, of a printed circuit board according to the fifth embodiment;
Fig. 18C shows a top face, and diagonal view, of a printed circuit board according to the fifth embodiment;
Fig. 19 shows a top face of a printed circuit board according to a sixth embodiment;
Fig. 20A shows a side face of a printed circuit board according to the sixth embodiment;
Fig. 20B shows a side face of a printed circuit board according to the sixth embodiment;
Fig. 20C shows a side face of a printed circuit board according to the sixth embodiment;
Fig. 21 is a diagram showing a cross-section of an ultrasonic transducer array according to a preferred embodiment of the present invention;
Fig. 22A is a diagram exemplifying a wire;
Fig. 22B is a diagram showing a cross-section of a wire;
Fig. 23A is a diagram exemplifying a tooling used when plating a wire; and
Fig. 23B is a diagram exemplifying a plating method for a wire.

### Best Mode for Carrying Out the Invention

An electronic radial ultrasonic endoscope according to the present invention, which is one using a printed circuit board comprising a plurality of pads electrically connecting piezoelectric elements arrayed in a circular pattern, is configured to equip the pads of the printed circuit board extended at least to an outer circumference of the printed circuit board.

The electronic radial ultrasonic endoscope according to the present invention, which is one comprising a printed circuit board or a structure member adjacent thereto, is also configured to provide at least one pad of a different form or size among a plurality of pads equipped on the printed circuit board.

The electronic radial ultrasonic endoscope according to the present invention is further configured to apply a marking close to the pads on the printed circuit board electrically connecting to a piezoelectric element constituting a reference as well as on the top face or side face of the printed circuit board, or the side face of a structure body adjacent to the printed circuit board.

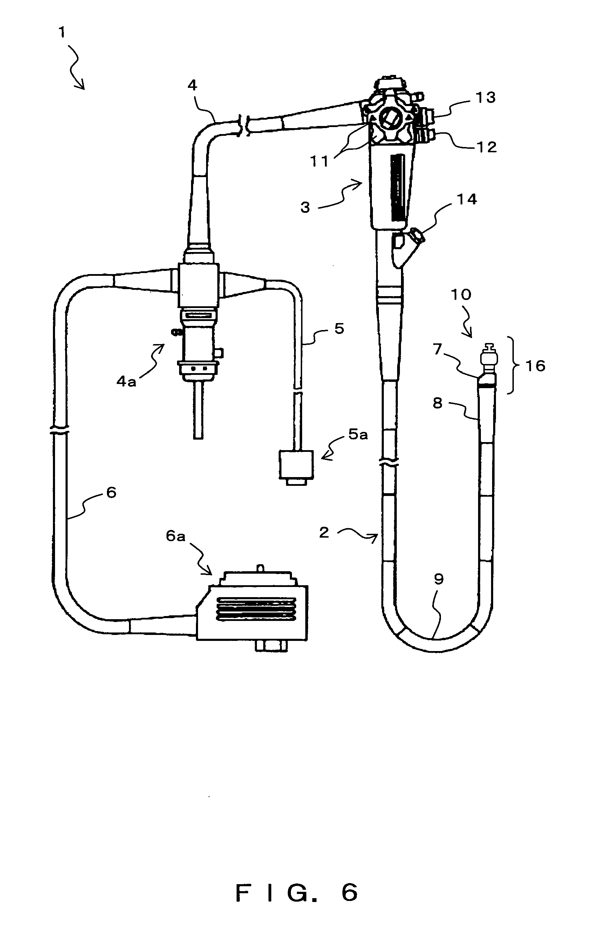

Fig. 6 is a diagram showing an external configuration of an ultrasonic endoscope according to the present invention. The ultrasonic endoscope 1 mainly comprises a slender insertion part 2 to be inserted into a body cavity, an operation part 3 positioned at the base end of the insertion part 2 and a universal cord 4 extending from the side of the operation part 3.

The base end part of the universal cord 4 is equipped with an endoscope connector 4a that is to be connected to a light source apparatus (not shown herein). From the endoscope connector 4a expended is an electric cable 5 that is to be freely detachably connected to a camera control unit (not shown herein) by way of an electric connector 5a, and also extended is an ultrasonic wave cable 6 that is to be freely detachably connected to an ultrasonic wave observation apparatus (not shown herein) by way of an ultrasonic wave connector 6a.

The insertion part 2 is structured by serially connecting, in order from the head side, a hard head part 7 formed by a hard plastic member, a curve part 8 positioned at the end of the hard head part 7 and allowing a free curving, a flexible tube part 9 positioning itself at the rear end of the curve part 8, extending to the head part of the operation part 3, featuring itself as small diameter and slender and possessing flexibility. And the head side of the hard head part 7 is equipped with an ultrasonic transducer 10 arraying a plurality of piezoelectric elements for emitting and receiving an ultrasonic wave.

The operation part 3 is equipped with such parts as an angle knob 11 for controlling a curvature of the curve part 8 in a desired direction, an air/water supply button 12 for carrying out an air or water supply operation, a suction button 13 for carrying out a suction operation, a treatment instrument insertion opening 14 for providing an entrance for introducing a treatment instrument into a body cavity.

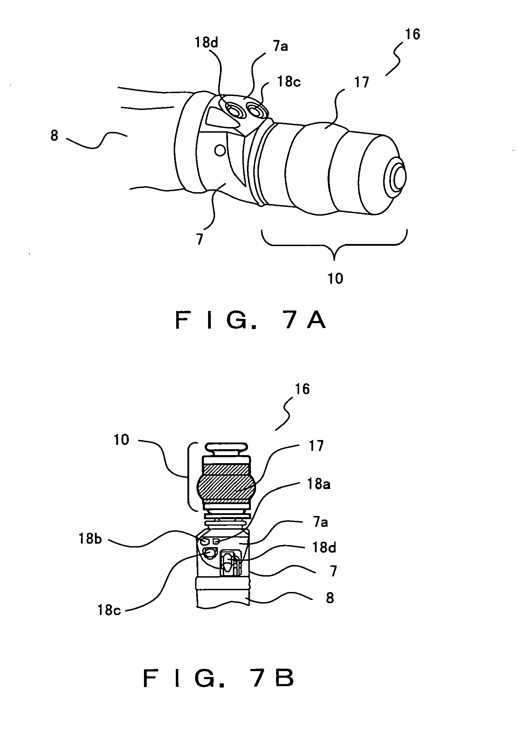

Fig. 7 is an enlarged diagram of an insertion part head 16 of the ultrasonic endoscope 1 shown in Fig. 6. Fig. 7A shows a diagonal external view diagram and Fig. 7B shows an external configuration diagram. The insertion part head 16 is equipped with the ultrasonic transducer 10 enabling an electronic radial scan. The ultrasonic transducer 10 is covered with a material forming an acoustic lens (i.e., an ultrasonic emission and reception unit) 17. The hard head part 7 is featured with an inclination part 7a. The inclination part 7a is equipped with an illumination lens 18b constituting an illumination optical part for emitting an illumination light to an observation region, an object lens 18c constituting an observation optical part for capturing an optical image of an observation region, a suction-cum-forceps entrance 18d that is an opening for sucking a cutoff region and projecting a treatment instrument, and an air/water supply entrance 18a that is an opening for supplying air and water.

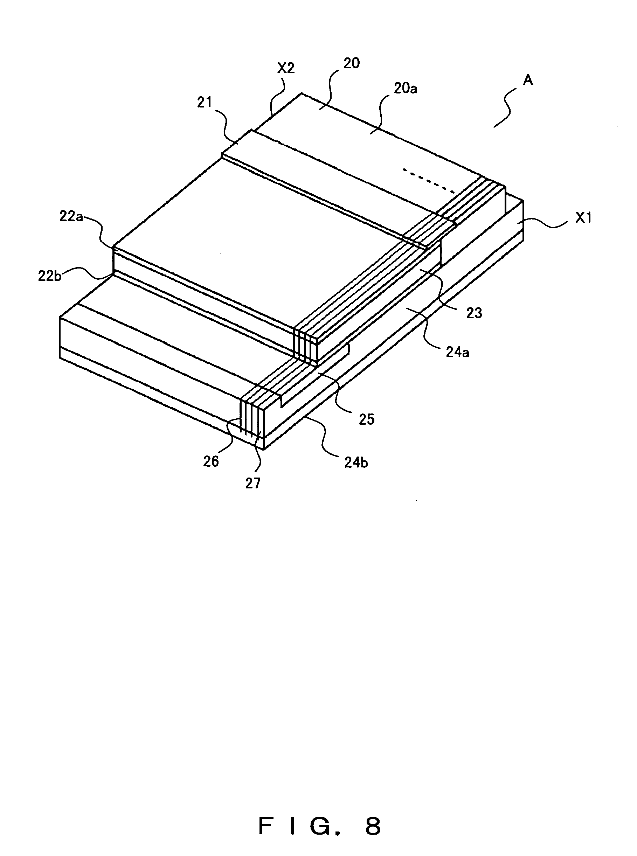

Fig. 8 is a diagram showing a production process (part 1) of an ultrasonic transducer. Referring to Fig. 8, when forming an ultrasonic transducer 10, first to make is a structure body A constituted by a board 20, a conductive body 21, electrodes 22 (i.e., 22a and 22b), a piezoelectric element 23, acoustic matching layers 24 (i.e., a first acoustic matching layer 24a and a second acoustic matching layer 24b), a conductive resin 25 and grooves 26. Now a description is of a manufacture of the structure body A.

The first step forms the second acoustic matching layer 24b followed by forming the first acoustic matching layer 24a. The next forms the grooves in the first acoustic matching layer 24a by using a dicing saw (i.e., a precision shearing machine) for example and pours a conductive resin 25 into the grooves. The next joins the piezoelectric element 23 that is featured with the electrode layers 22a and 22b on both of the opposite primary surfaces, followed by mounting a board 20 on the side of the piezoelectric element 23. An electrode layer 20a is featured on the surface of the board 20. The next mounts the conductive body 21 for electrically conducting between the electrode layer 20a and electrode layer 22a.

The next cuts in the structure body A formed as described above by using a dicing saw, thereby forming a plurality of grooves (diced grooves) 26 each of which is tens micrometers wide. The groove width is preferably 20 to 50 micrometers. The cut-in into the structure body A in this event is to leave tens micrometers thick of only the second acoustic matching layer 24b uncut. Approximately 200 pieces of such grooves 26 are formed. Here, the divided individual transducer is called an transducer element 27.

Note that the above description is on a two-layer matching according to the present embodiment and a material for the first acoustic matching layer 24a preferably uses an epoxy resin containing a filler such as alumina and titania (TiO₂), and that for the second acoustic matching layer 24b preferably uses an epoxy resin not containing a filler. Comparably, in the case of a three-layer matching, a material for the first acoustic matching layer preferably uses such as carbon or an epoxy resin containing a machinable ceramics, filler or fiber, that for the second acoustic matching layer preferably uses an epoxy resin containing a very small amount of filler such as alumina or titania (i.e. , lower content ratio as compared to the case of the two-layer matching), and that for the third acoustic matching layer preferably uses an epoxy resin not containing a filler.

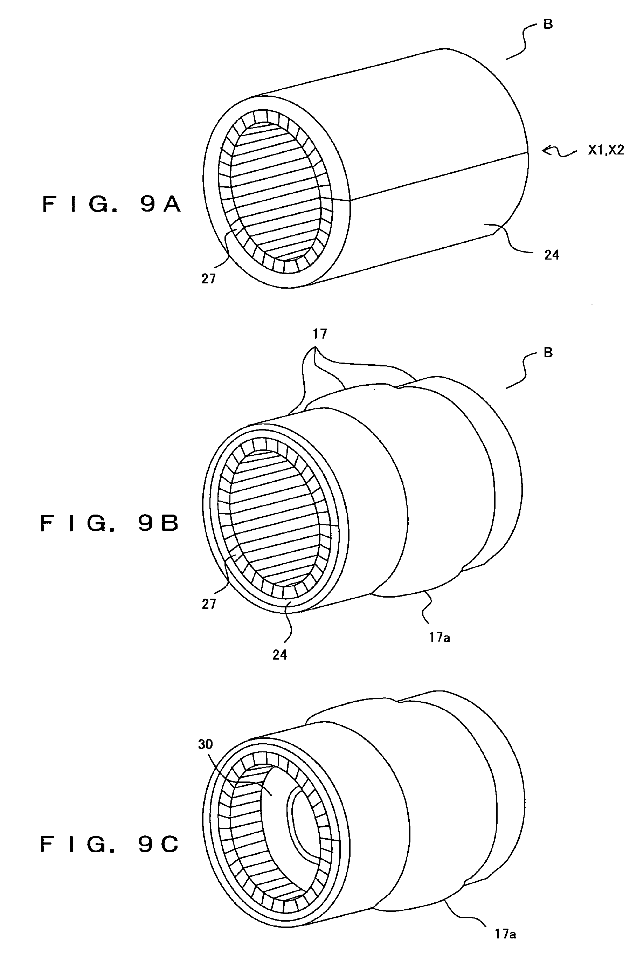

The next forms the structure body A in a cylindrical form by curving it so as to have the side faces X1 and X2 of the layered body are opposite to each other as shown in Fig. 9A.

The next forms the acoustic lens 17 on the cylindrical surface as shown in Fig. 9B (the resultant is named as a structure body B hereinafter). Alternatively, the acoustic lens 17 may be produced by combining a prefabricated single body of an acoustic lens with a cylindrically shaped structure body A, or the acoustic lens 17 may be produced by inserting a cylindrically shaped structure body A into a mold, followed by injecting an acoustic lens material into the mold. Note that it is a lens part 17a that actually functions as acoustic lens.

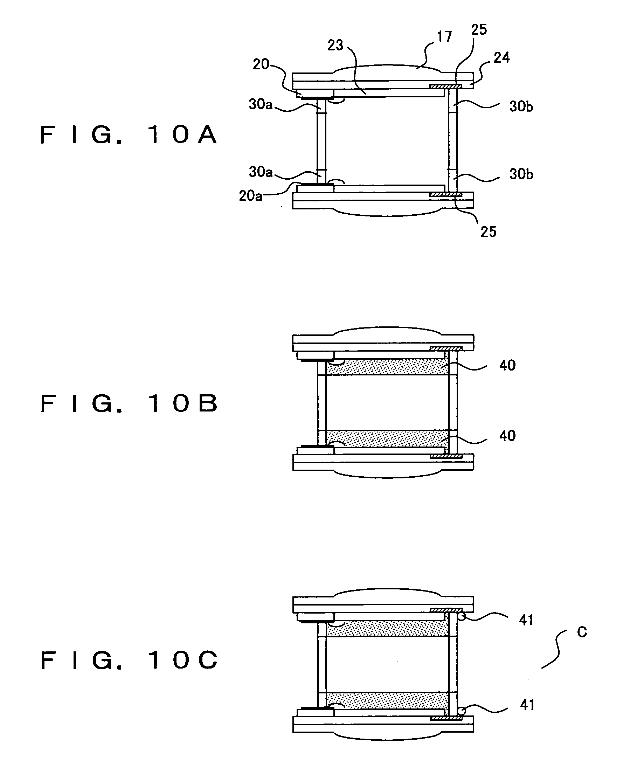

The next mounts a circular structure member 30a in the inside, and a little deeper than the opening, of the structure body B, as shown in Fig. 9C. The mounting in this event is such that the structure member 30a is located on the board 20 (refer to Fig. 10A). Likewise, a structure member 30b is mounted in the opening on the other side. The mounting in this event is such that the structure member 30b is located on the conductive resin 25 (refer to Fig. 10A).

Fig. 10 shows a cross-section of the structure body B on which the structure members 30 are mounted. After assembling the structure members 30 (i.e., 30a and 30b) (refer to Fig. 10A) as shown in Fig. 9C, fills in between the structure members 30a and 30b with a backing material 40 (refer to Fig. 10B). The backing material uses a gelatinous epoxy resin mixed with a filler that is made of alumina. The filling is followed by mounting a conductive body (cupper wire) 41 on the conductive resin 2-5 (refer to Fig. 10C) (the structure member produced as shown in Fig. 10 is named as a structure body C hereinafter).

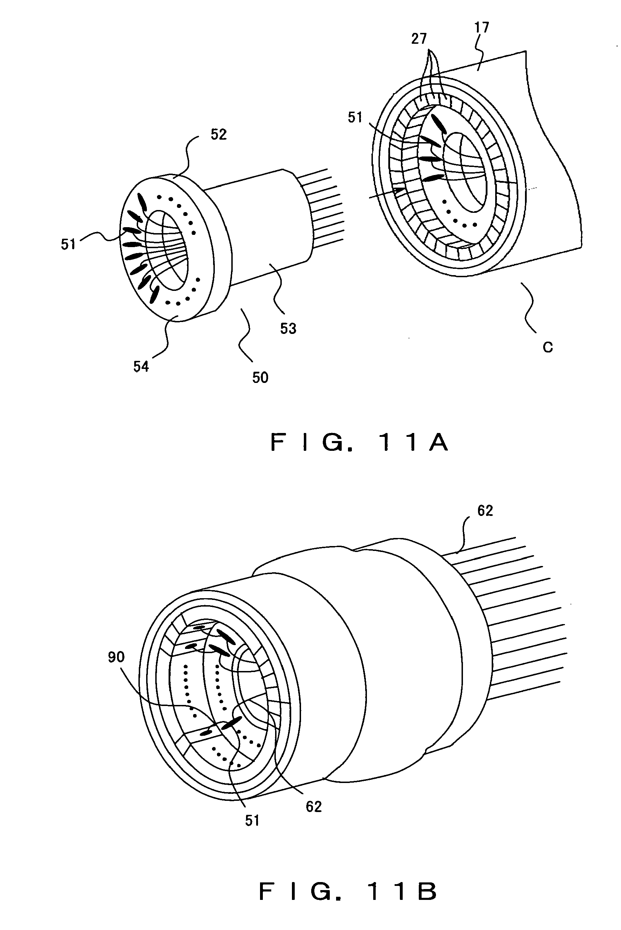

The next inserts a cylindrical structure member 50 from one opening side (i.e., the side equipped with the board 20) of the structure body C as shown in Fig. 11A. The cylindrical structure member 50 is constituted by a cylindrical part 53 and a circular flange 52 featured at one end thereof. The face of the flange 52 is equipped with a printed circuit board 54 on the surface of which are provided with tens to hundreds of electrode pads 51. Further, a bundle of cables 62 is led through the inside of the cylindrical structure member 50, with the tips of the individual cables 62 being soldered to the respective electrode pads 51 (that is, the cables 62 are connected by soldering in the inside (i.e., the center direction of circle) of the electrode pads 51). Note that the cables 62 usually use a coaxial cable for a noise reduction.

The cylindrical structure member 50 is made of an insulation material (e.g., an engineering plastic). The insulation materials include polysulfone, polyether imide, polyphenylene oxide, epoxy resin and such. The surface of the cylindrical part 53 is plated with a conductive material.

As the cylindrical structure member 50 connected with the cables 62 with wires is inserted into the structure body C, the flange 52 part of the cylindrical structure member 50 runs into the structure member 30 of the structure body C, thus fixing a position of the cylindrical structure member 50 in the inside of the structure body C and positioning internally in the transducer.

Fig. 11B shows a state of connecting the outer parts of the electrode pads 51 (i.e., the electrode pad part in the outer circumference direction of circle) to the electrode layer 20a of the transducer element 27 by using wires 90 after the cylindrical structure member 50 is inserted and positioned.

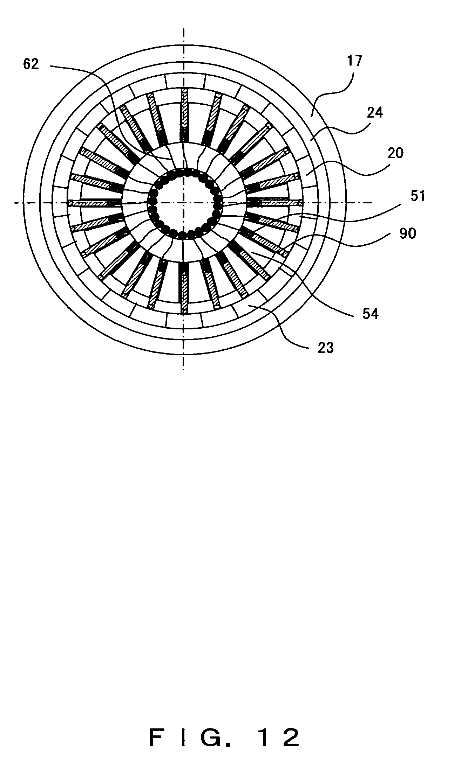

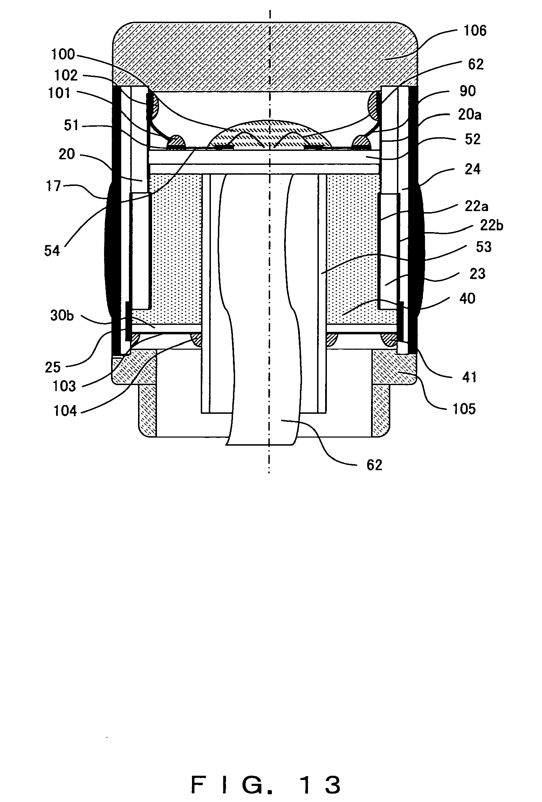

Fig. 12 shows an upper cross-sectional diagram of the head of the electronic radial ultrasonic endoscope shown in Fig. 11B; and Fig. 13 shows a side cross-sectional diagram of the head of the electronic radial ultrasonic endoscope shown in Fig. 11B. As described above, a plurality of coaxial cables 62 exists in the inside of the insertion part of the electronic radial ultrasonic endoscope. The coaxial cables 62 are electrically connected to the respective wires 90 by way of the pads 51 of the printed circuit board 54 and solder 101.

The coaxial cable 62 is potted by a resin 100. The wire 90 is connected to the board 20 that comprises an electrode layer 20a on the surface by way of a solder 102. The electrode layer 20a is electrically connected to the piezoelectric element 23 that comprises an electrode layer 22a on the surface.

Meanwhile, an electrode opposite the electrode layer 22a of the piezoelectric element 23 is the electrode layer 22b. The electrode layer 22b is electrically connected to the cylindrical part of the cylindrical structure member 50 by way of the conductive resin 25, cupper foil 103 featured on the structure member 30b and conductive adhesive 104.

The electrode layer 22a side of the piezoelectric element 23 is provided with the backing material 40, and the surface of the acoustic matching layer 24 is featured with the acoustic lens 17. The head of the transducer configured as described above is equipped with a head structure member 106, and a connection part with the hard head part 7 of the endoscope is equipped with a structure member 105.

The following shows preferred embodiments of such an electronic radial ultrasonic transducer according to the present invention.

### <First Embodiment>

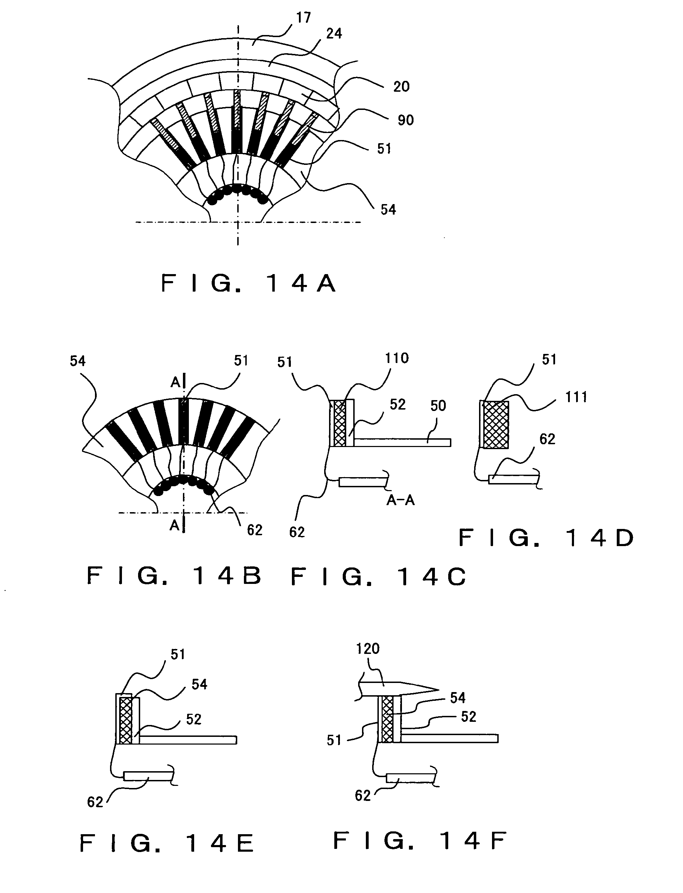

Fig. 14 shows a top face, and a side cross-section, of a printed circuit board 54 according to the present embodiment. Figs. 14A and 14B are abbreviated diagrams of Fig. 12. Figs. 14C, 14D, 14E and 14F are cross-sectional diagrams of the cylindrical structure member 50 when it is cut along the cutting line A - A.

As shown in Figs. 14A and 14B, the printed circuit board 54 is equipped on the front surface of the flange 52 of the cylindrical structure member 50 in a donut shape. The electrode pads (simply named as pads hereinafter) 51 featured on the printed circuit board 54 are radially extended to the edge part (i.e., the outer circumference side surface) of the printed circuit board 54.

Note that the printed circuit board 54 may be configured as a combination of a flexible board 110 with the flange 52 surface of the cylindrical structure member 50 as shown in Fig. 14C, or as a combination of a rigid board 111 with the flange 52 surface of a cylindrical structure member as shown in Fig. 14D. Moreover, the pad 51 may be configured to be equipped so as to cover the outer circumferential side surface of the printed circuit board 54 as shown in Fig. 14E.

The printed circuit board 54 according to the present embodiment provides the function as follows. First, provided is to enable a probe 120 for continuity check to contact with the side surface of the pad in place of the top surface thereof (refer to Fig. 14F). Therefore, the continuity between the pads 51 and coaxial cables 62 can be validated without causing damage to the pads 51 or the electrical connection part of the pads 51 and coaxial cables 62.

In the case of providing the pads 51 extended to the outer circumferential side surface of the printed circuit board 54, even if the printed circuit board 54 is cut to minimize the diameter thereof on an as required basis, the pads 51 always exist on the outer circumferential side surface of the post-cut printed circuit board 54. Therefore, even if the printed circuit board 54 is cut to a discretionary diameter in accordance with a diameter of the board 20, that is, an array diameter (i.e., a diameter of the opening of the structure body C) of the piezoelectric element 23, the pads 51 always exist on the outer circumferential side surface of the printed circuit board 54.

Also, the provision of the pads 51 extended to the outer circumferential side surface of the printed circuit board 54 makes it possible to widen a distance between the pads 51.

The above described configuration makes it possible to prevent a degradation of quality. This also makes it possible to easily produce a printed circuit board that electrically connects to a piezoelectric element being arrayed in a various diameters, thereby improving production efficiency. Also enabled is an easy touch of a continuity check-use probe 120 with a specific pad at the time of continuity validation, thus improving a workability.

### <Second Embodiment>

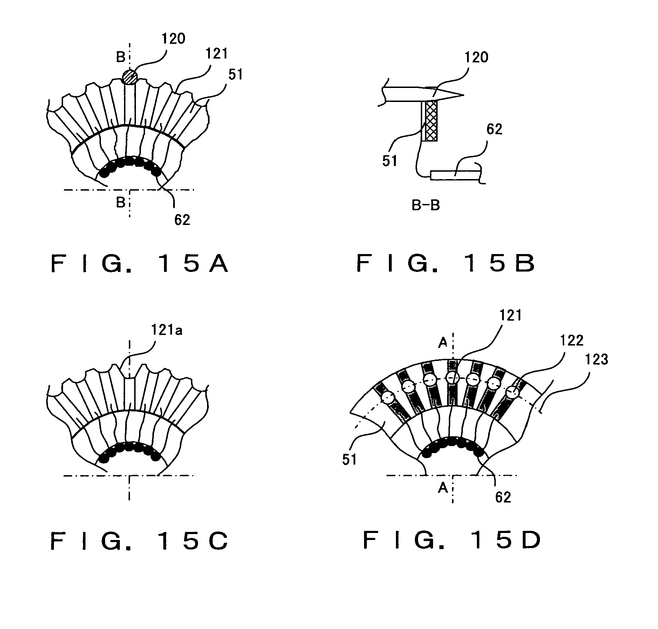

Fig. 15 shows a top face, and a side cross-section, of a printed circuit board 54 according to the present embodiment. Figs. 15A and 15C are abbreviated diagrams of the top face of the printed circuit board 54. Fig. 15B is a cross-sectional diagram of the printed circuit board 54 when it is cut along the cutting line B - B. Fig. 15D is a diagram for describing a forming of a positioning groove.

As shown in Figs. 15A and 15B, the outer circumferential side surface of the printed circuit board 54 featured with a pad 51 is featured with positioning grooves 121 so as to enable a positioning of the head of the continuity check-use probe 120.

Note that the size or form of a positioning groove 121a at one place or a plurality of places among the positioning grooves 121 may be changed as shown in Fig. 15C.

Means for featuring the pads 51 with the positioning grooves 121 is as follows. As an example, a plurality of pads 51 is formed on the top surface of the printed circuit board 54 as shown in Fig. 15D, followed by providing through holes 122 in a part contacting with the pads 51 and cutting the printed circuit board 54 at a cutting position 123 on the center line of the through holes 122. An alternative method is to punch out by using a tooling (i.e. , a cutout punch (not shown herein)) of the same form as the printed circuit board 54 having the positioning grooves 121 as an outer circumferential side surface. Or a laser (not shown herein) may be used for processing the outer side surface of the pads 51.

The printed circuit board 54 according to the present embodiment provides the following functions. First, the use of the positioning grooves 121 enables the continuity check-use probe 120 to touch with a specific pad 51 securely and smoothly. It also enables a use of a positioning groove 121 as a marker when working on wiring the coaxial cable 62 and wire 90 to a pad 51 or working on housing an transducer module into an endoscope after the wiring work.

The above described configuration enables an efficient validation of electrical continuity and thus an improvement of workability. Also enabled is a prevention of a wrong wiring and a mis-assembly at the time of housing, thus improving a production yield.

### <Third Embodiment>

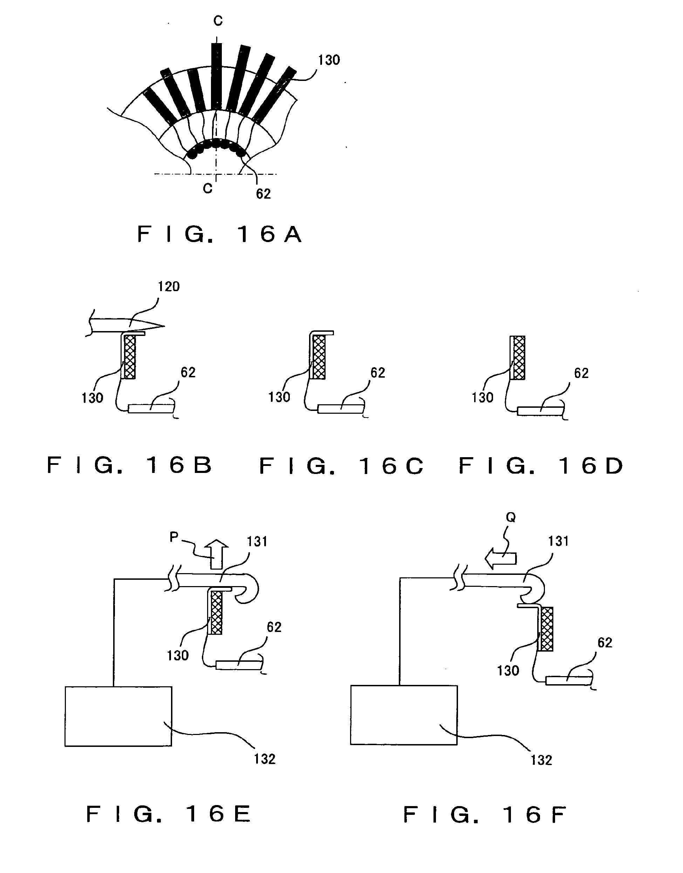

Fig. 16 shows a top face, and a side cross-section, of a printed circuit board 54 according to the present embodiment. Fig. 16A is an abbreviated diagram of a top face of the printed circuit board 54. Figs. 16B through 16F are cross-sectional diagrams of the printed circuit board 54 when it is cut along the cutting line C - C.

The printed circuit board 54 according to the present embodiment is equipped with pads 130 projecting into the air (which is called as a flying lead structure) by extending the pads farther than the end part (i.e., the outer circumference part) of the printed circuit board 54 as shown in Fig. 16A. And the printed circuit board 54 is equipped with a plurality of the pads 130.

The next bends the pads 130 on the side surface side of the printed circuit board, followed by validating an electrical continuity by using the continuity check-use probe 120 as shown in Fig. 16B. If there is no continuity, the pad 130 is left bent as shown in Fig. 16C. Contrarily, if there is continuity, the pad 130 is cut off at the end part (i.e., the outer circumferential side surface) of the printed circuit board 54 as show in Fig. 16D.

Note that the tip of a continuity check-use probe 120 may be a probe 131 formed as a hook form as shown in Fig. 16E. If the tip of the probe 131 is in a hook form, continuity is validated after bending the pad 130 on the side surface of the printed circuit board. If there is continuity in this event, a position control system 132, that is, an apparatus for biasing the tip of the probe 131 against a predetermined position, operates, thereby moving the probe 131 in the direction of the arrow P as shown in Fig. 16E without touching the pad 130, resulting in the pad 130 remaining as being bent.

Contrarily, if there is no continuity, the position control system 132 operates, thereby moving the probe 131 in the direction of the arrow Q, the hooked tip of the probe 131 catching the bent part of the pad 130 and bending it over as shown in Fig. 16F. This makes it easier to distinguish a presence or absence of continuity.

The printed circuit board 54 according to the present embodiment provides the following functions. First provision is to enable a discernment of a spot of no continuity at a glance. Moreover, the use of the probe 131 comprising a hook-formed tip enables an easier discernment of a presence or absence of continuity.

The above described configuration eliminates a missed or double work in a validation of continuity, thus improving reliability of a product.

### <Fourth Embodiment>

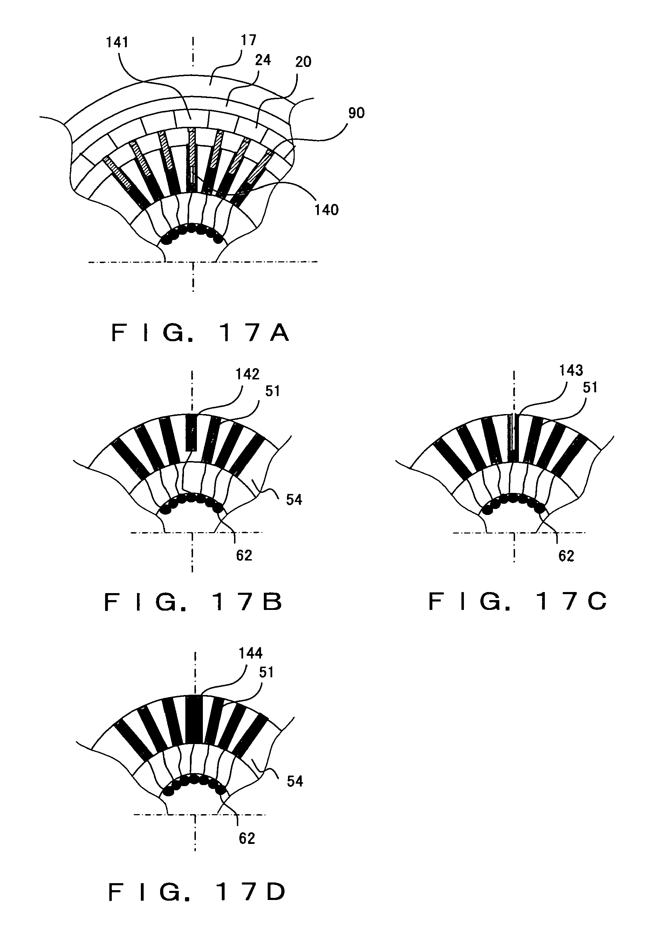

Fig. 17 shows a top face of a printed circuit board 54 according to the present embodiment. As shown in Fig. 17A, the printed circuit board 54 is equipped with at least one pad 140 of a different form or size (the configuration shown in Fig. 17A is equipped with a slit in the longitudinal direction of the pad) among a plurality of pads equipped on the printed circuit board 54.

As an example, a pad 142 of a shorter length may be equipped as shown in Fig. 17B. Alternatively, a wide pad 144 (i.e., a size in the shorter direction) may be equipped as shown in Fig. 17D. As another example, a pad 143 comprising a slit may be equipped among a plurality of pads equipped on the printed circuit board 54 as shown in Fig. 17C.

The printed circuit board 54 according to the present embodiment provides the following function. It is possible to use the pad 140 of a different form or size as a marker for positioning. Therefore, when connecting a coaxial cable 62, which is required to electrically connect to a piezoelectric element (not shown herein) constituting a reference, to a signal board 141, which is electrically connected to the piezoelectric element (not shown herein), an intervention of a pad 140 (i.e., 142, 143 or 144) of a different form or size prevents a wrong connection between the piezoelectric element and cable.

In summary, a production yield of a product is improved.

### <Fifth Embodiment>

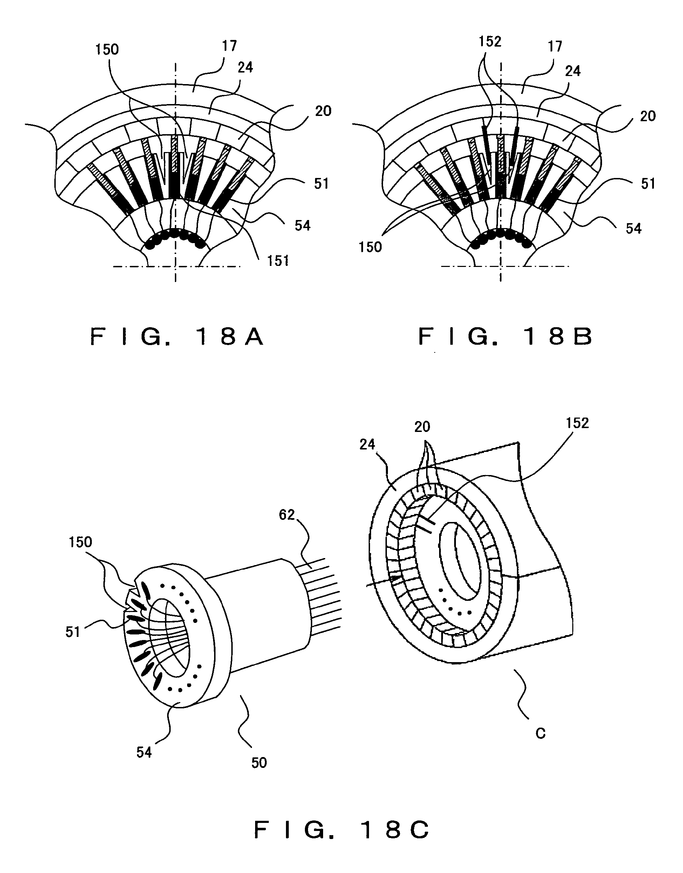

Fig. 18 shows a top face of a printed circuit board 54 according to the present embodiment. The printed circuit board 54 is featured with notches 150 in the one positioned on both sides of either one pad 151 among a plurality of pads 51 equipped on the printed circuit board 54 as shown in Fig. 18A.

A configuration may be such that a shim (i.e., a gap adjustment component) 152 is joined to each of the notches 150 on a surface of a structure member 30a that is positioned in a gap on both of the adjacent sides of a board 20 electrically connecting to a piezoelectric element (not shown herein) constituting a reference as shown in Fig. 18B, followed by inserting a cylindrical structure member into the structure body C as inserting the notch 150 into the shim 152 (refer to Fig. 18C).

The printed circuit board 54 according to the present embodiment provides the following function. The pad 51 equipped between the notches 150 is electrically connected to the board 20 connecting to the piezoelectric element constituting a reference by way of the printed circuit board 54. In this event, the notches 150 can be used as a positioning mechanism with regard to the piezoelectric element constituting a reference.

The above configuration makes it possible to prevent a wrong connection between the piezoelectric element and coaxial cable 62, improving a yield of a product. Also a connection work is enabled to carry out easily without requiring a minute adjustment of a position, thus improving workability.

### <Sixth Embodiment>

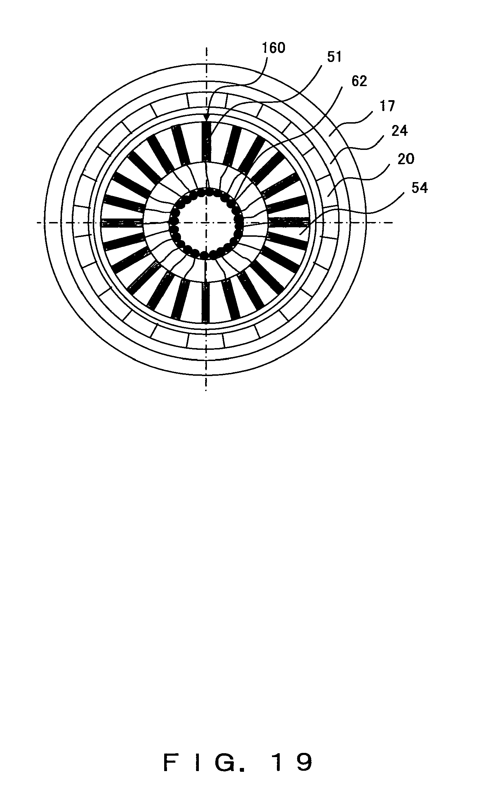

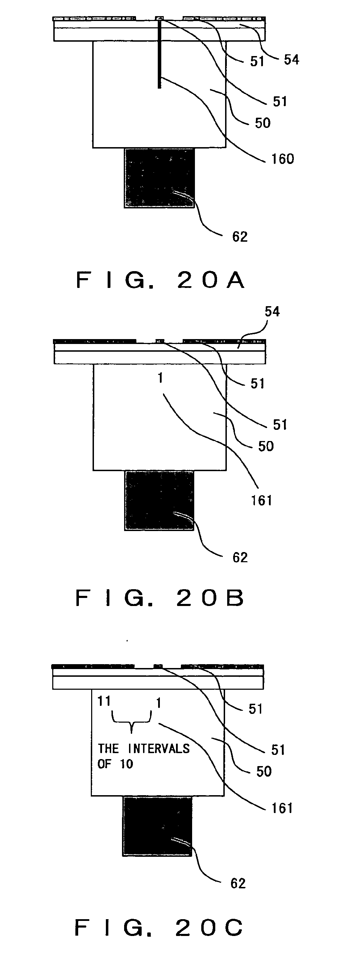

Fig. 19 shows a top face of a printed circuit board 54 according to the present embodiment. Fig. 20 shows a side face of the printed circuit board 54 according to the present embodiment. The printed circuit board 54 according to the present embodiment is configured such that the printed circuit board 54 or a cylindrical structure member 50 adjacent thereto is equipped with a marking 160. Equipment of the marking 160 in a close proximity to a specific piezoelectric element makes it easy to identify the piezoelectric element.

The marking 160 is provided in a close proximity to a pad 51 that is electrically connected to a piezoelectric element (not shown herein) constituting a reference as shown in Figs. 19 and 20A. The marking 160 is provided on the top or side surface of the printed circuit board 54, or the side surface of the cylindrical structure member 50 by employing a laser for example.

The marking 160 may be a numerical marking 161 as shown in Fig. 20B. In such a case, assuming that the number of a piezoelectric element (not shown herein) constituting a reference is "number 1" for example, then "1" is marked in the vicinity of a pad 51 of the printed circuit board 54 electrically connecting to the "number 1" element.

Furthermore, the numerical marking 161 may be provided in a plurality as show in Fig. 20C. In such a case, the numerical marking 161 is put down in intervals of ten, e.g., 1, 11, 21 and so on, corresponding to the piezoelectric elements. The numerical marking 161 is provided in a close proximity of the corresponding pad 51 as in the case of Fig. 20B.

The printed circuit board 54 according to the present embodiment provides the following function. When housing a module, in which the piezoelectric elements and coaxial cables 62 are connected together by way of the printed circuit board 54, in the head hard part 7, the marked spot can be used as a marker for positioning against the head hard part 7.

The configuration as described above improves an assembly workability of a product. Also enabled is a correlation between an optical image and an ultrasonic image securely, thereby improving a quality of the product.

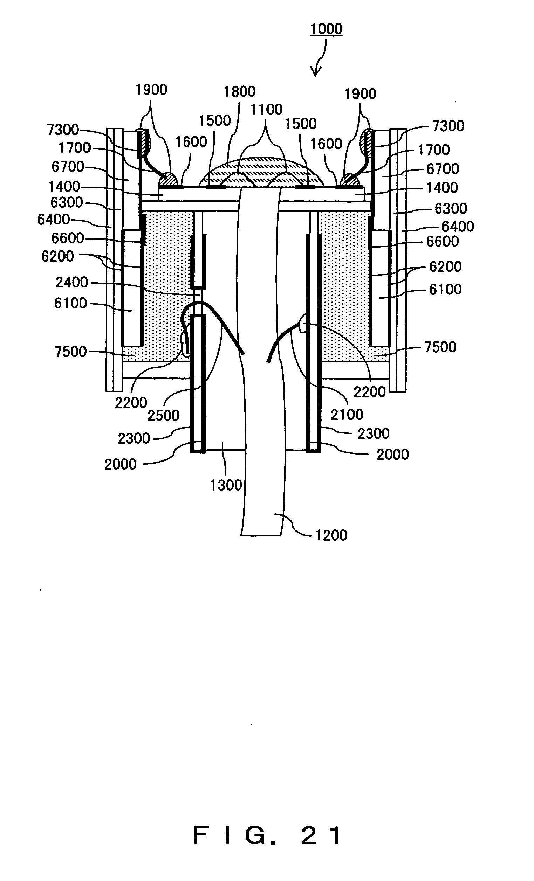

Fig. 21 is a diagram showing a cross-section of an ultrasonic transducer array according to a preferred embodiment of the present invention. Note that the same component sign is assigned to the same comprisal as one shown in Figs. 4A and 4B.

The ultrasonic transducer array 1000 (corresponding to the ultrasonic transducer 10 described above) shown by Fig. 21 is a radial type ultrasonic transducer array and equipped in an ultrasonic endoscope (corresponding to the above described ultrasonic endoscope 1) for example.

The ultrasonic transducer array 1000 comprises a piezoelectric element 6100, electrodes 6200, a first acoustic matching layer 6300, a second acoustic matching layer 6400, a conductive body 6600, a substrate 6700 and a backing material 7500. Assumption is that the piezoelectric element 6100, electrodes 6200, first acoustic matching layer 6300, second acoustic matching layer 6400, conductive body 6600 and substrate 6700 are commonly featured with grooves, thereby forming a plurality of ultrasonic transducers (corresponding to the above described transducer elements 27).

The ultrasonic transducer array 1000 also comprises a cylindrical structure member 1300 for leading a cable 1200, which is configured by bundling individual signal wires 1100 electrically connected to a plurality of ultrasonic transducers respectively, through the inside of the ultrasonic transducer array 1000.

The head part of the cylindrical structure member 1300 is equipped with a board 1400 on which a plurality of pads 1500 corresponding to respective signal wires 1100 are equipped.

The individual pads 1500 are electrically connected to a plurality of pads 1600 respectively by way of wiring patterns on the board 1400.

The ultrasonic transducer array 1000 also comprises a plurality of wires 1700 (corresponding to the above described wires 90) for electrically connecting the individual pads 1600 to the respective conductive layers 7300 equipped on the respective substrates 6700 of individual ultrasonic transducers.

As an example, if the ultrasonic transducer array 1000 is constituted by 200 pieces of ultrasonic transducers, then provided are 200 pieces of pads 1500, pads 1600, wires 1700 and conductive layers 7300, respectively.

The individual signal wires 1100 and pads 1500 are electrically connected to each other respectively, and fixed with a potting resin 1800. One respective ends of the individual wires 1700 are electrically connected to the respective pads 1600 by virtue of different kind of metal (which is described later) 1.900 and the other ends of the individual wires 1700 are electrically connected to the respective conductive layers 7300 by virtue of the different kind of metal.

By the above, the individual ultrasonic transducers are electrically connected to the respective signal wires by way of the pads 1500, pads 1600, wires 1700, conductive layers 7300, conductive bodies 6600 and electrodes 6200 on one side.

Meanwhile, an inside surface 2000 of the cylindrical structure member 1300 is plated with a conductive metal and connected to a ground (GND) wire 2100 extended to the cylindrical structure member 1300 by going into the inside of the cable 1200 by using a solder 2200. Also, an outside surface 2300 of the cylindrical structure member 1300 is plated with a conductive metal and is connected, by a solder 2200, to a GND wire 2500 that is extended to the cylindrical structure member 1300 by going in the inside of the cable 1200 and that is led through a hole 2400 that is put in the side wall of the cylindrical structure member 1300. Meanwhile, the electrode 6200 on the other side is assumed to be electrically connected to the outside surface 2300 of the cylindrical structure member 1300.

The characteristic of the ultrasonic transducer array 1000 lies in configuring each of the wires 1700 by coating the entirety of a substrate of the wire 1700 with a different kind of conductive metal 1900 that is different from the substrate in a predetermined thickness, and melting the different kind of metal at both ends of each wire 1700 by using a soldering iron or the like, thereby electrically connecting the individual ultrasonic transducers to the respective signal wires 1100.

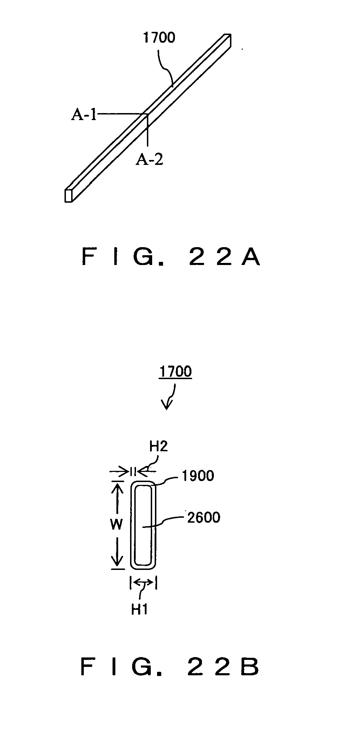

Fig. 22A is a diagram exemplifying the wire 1700; and Fig. 22B is a diagram showing a cross-section A1 - A2 of the wire 1700 shown in Fig. 22A.

A conductive wire constituting a substrate 2600 of the wire 1700 is formed as a plate in the size of 0.1 mm of the width Wand 0.025 mm of the thickness H1, namely a rectangular wire, with a material of the substrate 2600 being conceivably a good conductive metal such as cupper and silver as show in Fig. 22A and Fig. 22B. A form of the substrate 2600 may be a round wire in lieu of being limited to the rectangular wire. If the rectangular wire is used for the substrate 2600, a contact area between the pad 1600 and wire 1700, and that between the wire 1700 and conductive layer 7300 can both be maintained to be large as compared to the case of using a round wire, thereby securing the connection between the pads 1600 and wires 1700, and between the wires 1700 and conductive layers 7300, thus resulting in improving reliability of wire harness part of the wires. Also, if the rectangular wire is used for the substrate 2600, a necessary allowable current can be obtained by the smallest possible cross-sectional area size, and it is therefore suitable to minimizing a diameter of the wire 1700 and a fine pitch wiring.

Meanwhile, the outer circumference of the substrate 2600 is coated with a different kind of metal 1900 of an approximately uniform H2 thick (e.g., 0.005 mm thick) for the entire length of the substrate 2600 as shown in Fig. 22B. Note that the different kind of metal 1900 is conceivably a metal of the main component being lead, tin, nickel or bismuth for example. The thickness of the different kind of metal 1900 is preferably of a thickness so as not to bridge with an adjacent wire 1700 when the different kind of metal 1900 is melted by using a soldering iron or the like.

The wire 1700 is configured by coating the substrate 2600 with the different kind of metal 1900 of a predetermined thickness as described above and therefore the individual ultrasonic transducer can be connected to the respective signal wire 1100 by a predetermined amount of the different kind metal 1900.

This configuration makes it possible to prevent an occurrence of a bridge at the wiring part of each wire 1700 and therefore it is possible to make the wiring work between the pads 1600 and wires 1700 and between the wires 1700 and conductive layers 7300 easy even if the individual ultrasonic transducers of the ultrasonic transducer array 1000 are arrayed in a small pitch.

Also, the coating of the entirety of the substrate 2600 with the different kind of metal 1900 enables a prevention of oxidization of the substrate 2600. This configuration improves an anticorrosion resistance of the wire 1700, stabilizing a quality of the ultrasonic transducer array 1000, and also improves a workability of wiring the wires 1700. Also, the wire 1700 configured by coating the entirety of the substrate 2600 with different kind of metal 1900 provides a particular benefit as wires used for the ultrasonic transducer array incorporated in an ultrasonic endoscope that is subjected to a frequent cleaning in the production process of the ultrasonic endoscope. Also, the wire 1700 configured by coating the entirety of the substrate 2600 with different kind of metal 1900 possesses a certain level of rigidity, making it possible to improve a workability of wiring work of the wires 1700.

Meanwhile, the end surfaces of both ends of the wire 1700 may be left uncoated with the different kind of metal 1900. The end surfaces of such configured wire 1700 are covered with a melted different kind of metal 1900 when connecting the wires 1700 to the pads 1600 or conductive layers 7300. This makes it possible to discern whether of not the pads 1600 and the wire 1700 or the wires 1700 and conductive layers 7300 are connected to each other by the different kind of metal 1900 easily just by confirming whether or not the end surfaces of the wire 1700 are covered with the different kind of metal 1900, thus enabling an improvement of a workability of wiring the wires 1700.

Also, the wire 1700 configured by coating the entirety of the substrate 2600 with the different kind of metal 1900 eliminates a necessity of judging whether or not a backup solder is provided for each wire 1700 unlike the case of a conventional technique, thereby making it possible to reduce an erratic wiring work for the wires 1700.

The next is a description of a plating method for coating the substrate 2600 with the different kind of metal 1900.

While a common plating method conceivably includes, for example, a wet plating (e.g., electroplating, chemical plating, (electroless plating), et cetera), a dry plating (e.g. , vacuum vapor deposition, sputtering method, ion plating method, et cetera), a chemical vapor deposition (CVD) plating, or a melt-plating (i.e., a dipping), a wet plating is preferable for coating the entirety of a substrate 2600 with a different kind of metal 1900 of an approximately uniform thickness as described above.

The following is a description of a method for coating a substrate 2600 with the different kind of metal 1900 by an electroplating method that is a wet plating method.

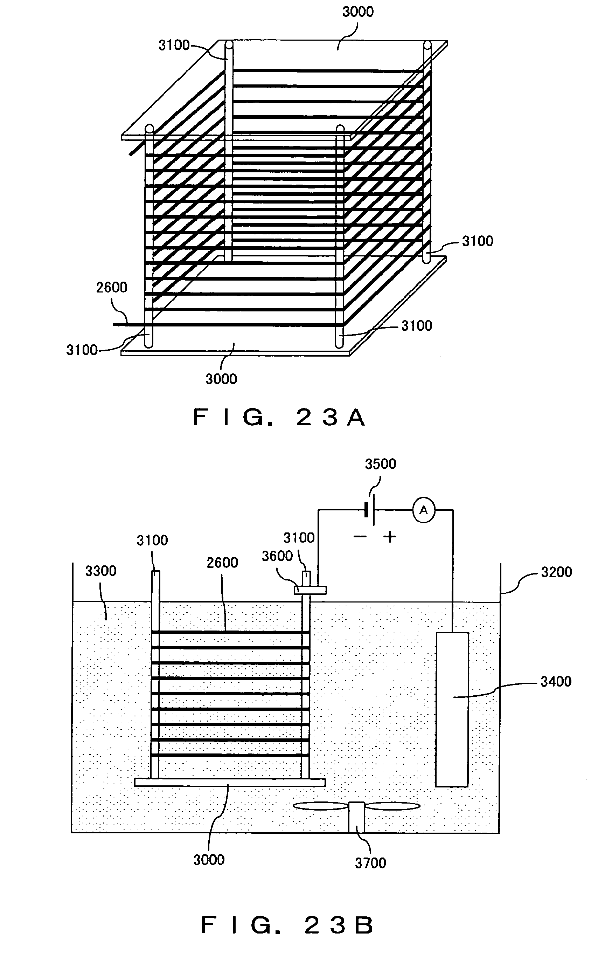

Fig. 23A is a diagram exemplifying a tooling used for plating a substrate 2600 with a different kind of metal 1900. Note that the same component sign is assigned to the same comprisal as one shown in Figs. 22A and 22B.

As shown in Fig. 23A, the fixing tool 3000 is structured by square plate members. While the material or size of the fixing tool 3000 is not particularly constrained as such, the plate members desirably have no metal part on the surface thereof for use in an electroplating method.

The four corners of the fixing tool 3000 are respectively equipped with metal bars 3100. Note that the number of the metal bars 3100 equipped in the fixing tool 3000 may be two or five or more, in lieu of being limited as shown.

Meanwhile, the substrate 2600 constituting the wire 1700 is wound around the four metal bars 3100 with certain intervals between.

By the substrate 2600 being wound around the metal bars 3100, the substrate 2600 can be coated with the different kind of metal 1900 in the state of extending the substrate 2600.

Fig. 23A exemplifies a configuration of one string of the substrate 2600 being wound around the four metal bars 3100; a plural number of the substrates 2600, however, may be wound around the four metal bars 3100 by maintaining certain intervals between them.

Fig. 23B is a diagram exemplifying an electroplating method that is a wet plating method. Note that the same component sign is assigned to the same comprisal as one shown in Fig. 23A. Also, a plating tank 3200 is assumed to be already filled with a plating fluid 3300.

The first step sinks an electrode plate 3400 and the substrate 2600 wound around the metal bars 3100 into the plating fluid 3300.

The next connects the electrode plate 3400 to a positive electrode of a power supply 3500 and also connects a terminal 3600 equipped at the head part of one of the metal bars 3100 to the negative electrode of the power supply 3500 on the outside of the plating fluid 3300. Also assumed is that the bottom of the plating tank 3200 is equipped with a stirrer apparatus 3700 and that the plating fluid 3300 is stirred thereby.

As an example, in the case of preparing a plating fluid 3300 with a composition of metallic component between 87% and 92% of tin with remainder being lead, using an electrode plate 3400 constituted by 85% tin and plating the substrate 2600 slowly by lowering the electric current to adjust the voltage value at one volt, then an 85% tin is coated on the substrate 2600.

Alternatively, as an example, in the case of preparing a plating fluid 3300 with a composition of metallic component between 87% and 92% of tin with remainder being lead, using an electrode plate 3400 constituted by 85% tin and plating the substrate 2600 by increasing the electric current, then an 87% to 92% tin is coated on the substrate 2600.

Or, the substrate 2600 may be coated with a lead-free different kind of metal 1900 by using the plating method shown by Fig. 23B. As an example, the substrate 2600 may be coated with a different kind of metal 1900 composed of a 100% tin or tin-cupper.

Then, the wire 1700 and shown in Figs. 22A and 22B is configured by cutting out the substrate 2600 coated with the different kind of metal 1900 from the metal bars 3100.

Such use of the electroplating method that is wet plating as a plating method for coating the substrate 2600 with the different kind of metal 1900 makes it possible to coat the different kind of metal 1900 of an approximately uniform thickness on the entirety of the substrate 2600. Adjusting of a metal component of the plating fluid 3300 and/or an electrolyzing time makes it possible to control a thickness of the different kind of metal 1900 to an approximately prescribed thickness.

This method enables the adjustment of the thickness of the different kind of metal 1900 so as not to allow a bridge with an adjacent wire 1700 when the different kind of metal 1900 is melted by using a soldering iron or the like for example, and therefore an occurrence of a bridge in a wiring part of all the wires 1700 can be prevented even if individual ultrasonic transducers are lined up in a very narrow pitch as in the case of the individual ultrasonic transducers used for an ultrasonic transducer array incorporated in an ultrasonic endoscope, thereby enabling an easy wiring work of the wires 1700 when electrically connecting the ultrasonic transducers to the respective signal wires 1100 by way of the wires 1700.

Note that the above described embodiment is configured to coating the substrate 2600 with the different kind of metal 1900 by means of an electroplating method that is wet plating, the substrate 2600, however, may be coated with a different kind of metal 1900 by employing a chemical plating method that is wet plating. In such a case of coating the substrate 2600 with the different kind of metal 1900 by means of a chemical plating method that is wet plating, adjustments of metallic component (such as palladium for example) in the plating fluid and/or time for immersing the substrate 2600 in the plating fluid make it possible to control the thickness of the different kind of metal 1900 to an approximately prescribed thickness.

As described above, also in the case of coating the substrate 2600 with the different kind of metal 1900 by means of a chemical plating method that is wet plating, the different kind of metal 1900 of an approximately uniform thickness can be coated on the entirety of the substrate 2600 and also a thickness of the different kind of metal 1900 can be controlled to an approximately prescribed thickness.

As described thus far, the present invention eliminates a possibility of damaging a pad, and a connection part between the pad and cable when validating an electric continuity by using a continuity validation-use probe.

It also enables a prevention of a wrong connection when electrically connecting piezoelectric elements to the respective pads of a printed circuit board that is connected by cables.

It also enables a prevention of an occurrence of a displacement between an optical image and an ultrasonic image.

The present invention is also contrived to melt a different kind of metal of a prescribed thickness coated on a substrate of a wire, thereby electrically connecting signal wires to respective ultrasonic transducers by way of the wire, and therefore the individual signal wires can be connected to the respective ultrasonic transducers by a predetermined amount of the different kind of metal respectively. This contrivance makes it possible to prevent an occurrence of a bridge in the wiring part of all wires, and therefore a wiring work can be made easy when connecting the signal wires to the respective ultrasonic transducers by way of the wires, even in the case of the individual ultrasonic transducers are arrayed in a small pitch.

## Claims

1. An electronic radial type ultrasonic transducer arraying a plurality of piezoelectric elements in a circular pattern for emitting and receiving an ultrasonic wave, storing cables corresponding to each of the piezoelectric elements for transmitting a drive signal driving each piezoelectric element in the inside of a cylindrical body formed thereby, forming electrode pads electrically connecting the cables and piezoelectric elements together on a printed circuit board, featuring a hole at the center of the printed circuit board in the planar direction for letting the cables go through, and equipping the printed circuit board on the cylindrical body, wherein
the electrode pads are formed on the top surface of the printed circuit board in a radial pattern and also exposed relative to an outer circumference of the printed circuit board.

2. The electronic radial type ultrasonic transducer according to claim 1, forming
a groove in a shorter direction of the outer circumference of the printed circuit board, of which a part contacts with the electrode pads.

3. The electronic radial type ultrasonic transducer according to claim 1, wherein
at least one electrode pad of the plurality thereof is in a different shape or size from other electrode pads.

4. The electronic radial type ultrasonic transducer according to claim 1, wherein
the printed circuit board related to both sides of at least one electrode pad of the plurality of electrode pads is equipped with a cutoff and a guide member is equipped at a predetermined position in the inside of the cylindrical body in order to guide the printed circuit board along the cutoff for installing the printed circuit board.

5. The electronic radial type ultrasonic transducer according to claim 1, providing
a marking on the top or side surface of the printed circuit board, or on a side surface of a support member supporting the printed circuit board, for identifying the electrode pad electrically connecting to a predetermined piece of the piezoelectric elements.

6. An ultrasonic endoscope comprising the electronic radial type ultrasonic transducer according to either one of claims 1 through 5.

7. An ultrasonic transducer array comprising a plurality of ultrasonic transducers continuously lined up and a plurality of signal wires electrically connected to the plurality of ultrasonic transducers respectively, comprising
a plurality of wires respectively equipped between the plurality of ultrasonic transducers and the plurality of signal wires for electrically connecting the plurality of ultrasonic transducers and the plurality of signal wires respectively, wherein
each of the plurality of wires is constituted by coating, in a predetermined thickness, the entirety of a metal constituting a substrate of the wire with a conductive metal that is different kind from the substrate, and
the different kind of metal is melted at both ends of the respective wires, thereby electrically connecting the plurality of signal wires to the plurality of ultrasonic transducers respectively.

8. The ultrasonic transducer array according to claim 7, wherein
the wire is configured by coating the entirety of the substrate with the different kind of metal of a thickness so as not to become bridged with an adjacent wire when the different kind of metals at both ends of the wires are respectively melted.

9. The ultrasonic transducer array according to claim 7, wherein
the substrate is constituted by cupper or silver, and
the different kind of metal is constituted by a metal of which the main component is lead, tin or bismuth.

10. The ultrasonic transducer array according to claim 7, wherein
the wire is configured by coating the entirety of the substrate with the different kind of metal by means of a wet plating.

11. The ultrasonic transducer array according to claim 7, wherein
the substrate is not covered with an insulator.

12. The ultrasonic transducer array according to claim 7, wherein
the substrate is a rectangular wire.

13. The ultrasonic transducer array according to claim 7, wherein
end surfaces of both ends of the wire is not coated with a different kind of metal.

14. A production method for a wire equipped between an ultrasonic transducer and a signal wire for electrically connecting the ultrasonic transducer and signal wire together for an ultrasonic transducer array that comprises a plurality of ultrasonic transducers continuously lined up and a plurality of signal wires electrically connected to the plurality of ultrasonic transducers respectively,
configuring the wire by coating the entirety of a substrate constituting a substrate of the wire with different kind of metal from the substrate by means of a wet plating.

15. The production method for a wire according to claim 14, wherein
the wet plating is an electroplating or chemical plating.

16. The production method for a wire according to claim 14,
applying a wet plating to the substrate by winding the substrate onto a plurality of rods equipped on a fixing tool.
